(19) Europäisches Patentamt
European Patent Office
Office européen des brevets

(11) Veröffentlichungsnummer : **0 546 993 A1**

(12) # EUROPÄISCHE PATENTANMELDUNG

(21) Anmeldenummer : **92810932.1**

(22) Anmeldetag : **01.12.92**

(51) Int. Cl.$^5$ : **C07D 401/12,** C08K 5/3492, C07D 401/14

(30) Priorität : **09.12.91 CH 3609/91**

(43) Veröffentlichungstag der Anmeldung :
**16.06.93 Patentblatt 93/24**

(84) Benannte Vertragsstaaten :
**AT BE CH DE ES FR GB IT LI SE**

(71) Anmelder : **CIBA-GEIGY AG**
**Klybeckstrasse 141**
**CH-4002 Basel (CH)**

(72) Erfinder : **Fuso, Francesco, Dr.**
**Bienenweg 8**
**CH-4106 Therwil (CH)**
Erfinder : **Reinert, Gerhard, Dr.**
**Weiherweg 1/7**
**CH-4123 Allschwil (CH)**

(54) **Wasserlöschliche Triazinderivate zur photochemischen und thermischen Stabilisierung von Polyamidfasermaterialien.**

(57) Beschrieben werden wasserlösliche Triazinderivate gemäss Formel (1). Die neuartigen Verbindungen stellen Vertreter der sterisch gehinderten Amine ("HALS"-Stabilisatoren) dar und eignen sich dazu, die thermische und photochemische Stabilität von ungefärbten und gefärbten Polyamid-Fasermaterialien zu erhöhen.

EP 0 546 993 A1

Die vorliegende Erfindung betrifft wasserlösliche Triazinderivate, Verfahren zur Herstellung dieser Verbindungen sowie ein Verfahren zur photochemischen und thermischen Stabilisierung von Polyamid-Fasermaterialien mit diesen Verbindungen.

Die neuen Verbindungen entsprechen der Formel

(1)

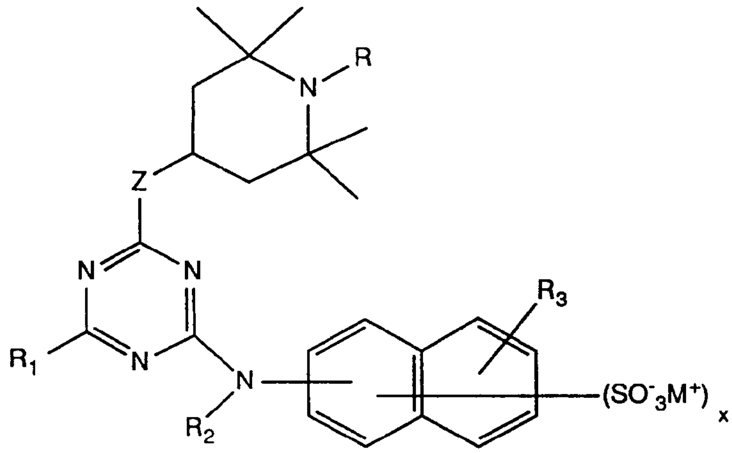

worin

R Wasserstoff; Oxyl; Hydroxy; $C_1$-$C_5$Alkyl; $C_3$-$C_5$Alkenyl; $C_1$-$C_5$Alkoxy; Acyl; oder Benzyl

$R_1$ Halogen; $C_1$-$C_5$Alkyl; Amino; $C_1$-$C_5$Alkoxy; $C_3$-$C_5$Alkenyloxy; Cycloalkoxy; nicht substituiertes oder im Phenylteil durch Halogen, $C_1$-$C_5$Alkyl, $C_1$-$C_5$Alkoxy, Carboxy, Carboxy-$C_1$-$C_5$Alkyl, Carbamoyl, Mono- oder Di-$C_1$-$C_5$-Acylamino oder Acyl substituiertes Phenoxy; Phenyl; Phenyl-$C_1$-$C_5$Alkyl; Phenylthio; Phenyl-$C_1$-$C_5$Alkylthio; Mono- oder Diphenyl-$C_1$-$C_5$Alkylamino; $C_1$-$C_5$Alkylthio; Cycloalkylthio; nicht substituiertes oder durch Hydroxy oder Carboxy substituiertes Mono- oder Di-$C_1$-$C_5$Alkylamino, wobei die Alkylkette durch ein Sauerstoffatom unterbrochen sein kann; Mono- oder Di-$C_3$-$C_5$Alkenylamino; nicht substituiertes oder durch $C_1$-$C_5$Alkyl substituiertes Mono- oder Dicycloalkylamino; nicht substituiertes oder durch $C_1$-$C_5$Alkyl,

Hydroxy oder Carboxy substituiertes 1-Azacycloalkyl; nicht substituiertes oder durch $C_1$-$C_5$Alkyl substituiertes Morpholino; einen Rest der Formel

(2)

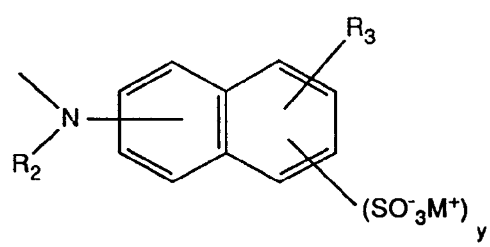

,

einen Rest der Formel

(3)

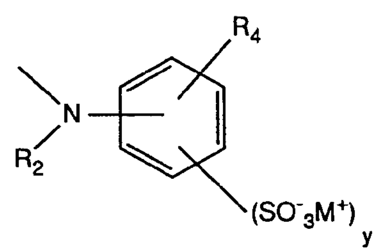

oder einen Rest der Formel

(4) 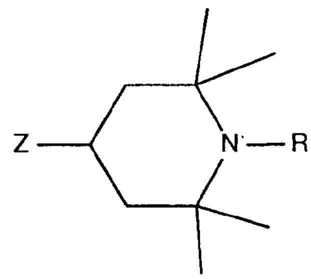

$R_2$ Wasserstoff; oder $C_1$-$C_5$Alkyl,
$R_3$ Wasserstoff; oder Hydroxy,
$R_4$ Wasserstoff; Halogen; $C_1$-$C_5$Alkyl; $C_1$-$C_5$Alkoxy; Carboxy; Carboxy-$C_1$-$C_5$Alkyl; Acyl; Carbamoyl; oder Mono- oder Di-$C_1$-$C_5$-Acylamino,
M gleich oder verschieden sein können und Wasserstoff; Alkalimetall; Erdalkalimetall; Ammonium; oder einen organischen Ammoniumrest der Formel $(C_1\text{-}C_4Alkyl)_n(H)_mN^+$,
Z -O-; oder -($NR_5$)-,
$R_5$ Wasserstoff; oder $C_1$-$C_5$Alkyl,
m 0 bis 3;
n 1 bis 4; und die Summe m + n = 4
x 1 oder 2 und
y 0 oder 1,bedeuten

und, wenn $R_1$ einen Rest der Formel (2) oder (3) bedeutet und y 1 ist, x in Formel (1) 1 ist.

$C_1$-$C_5$Alkyl steht beispielsweise für Methyl, Ethyl, n-Propyl, Isopropyl, n-Butyl, Isobutyl, sek.Butyl, tert.Butyl, Amyl oder Isoamyl, $C_1$-$C_5$Alkoxy für Methoxy, Ethoxy, Isopropoxy, Isobutoxy, tert.Butoxy oder tert.Amyloxy, $C_1$-$C_5$Alkylthio für Methylthio, Ethylthio, Propylthio oder Butylthio. Beispiele für Mono- und Di-$C_1$-$C_5$Alkylamino sind Dimethylamino, Diethylamino, Dipropylamino oder Methylethylamino, für Mono-oder Di-$C_1$-$C_5$-Acylamino Formyl-, Acetyl-, Propionyl-, Butyryl-, Diformyl-, Diacetyl-, Dipropionyl-, Dibutyryl- oder Formylacetylamino.

Cycloalkyloxy-, Mono- oder Dicycloalkylamino- und Cycloalkylthiogruppen weisen 4 bis 8, vorzugsweise 5 bis 7 Kohlenstoffatome auf. Beispiele für solche Gruppen sind Cyclobutyloxy, Cyclopentyloxy, Cyclohexyloxy, Methylcyclohexyloxy, Ethylcyclohexyloxy, Cycloheptyloxy, Cyclooctyloxy, Monocyclohexylamino, Dicyclohexylamino, Cycloheptylthio oder Cyclohexylthio. Als bevorzugte Cycloalkyloxygruppe kommt Cyclohexyloxy, als bevorzugte Cycloalkylaminogruppe Dicyclohexylamino und als bevorzugte Cycloalkylthiogruppe Cyclohexylthio in Frage.

$C_3$-$C_5$Alkenyl, $C_3$-$C_5$Alkenyloxy, Mono- oder Di-$C_3$-$C_5$Alkenylamino sind über ein gesättigtes Kohlenstoffatom gebundener Reste wie zum Beispiel Butenyl, Allyl, Butenyloxy, Allyloxy, Monobutenylamino, Monoallylamino, Diallylamino oder Dibutylamino. Bevorzugte Reste sind dabei Allyl, Allyloxy, Monallylamino und Diallylamino.

Phenyl-$C_1$-$C_5$Alkyl bedeutet beispielsweise Phenethyl, Phenylpropyl, Phenylbutyl oder vorzugsweise Benzyl. Beispiele für Phenyl-$C_1$-$C_5$-Alkoxy, Mono- oder Di(Phenyl-$C_1$-$C_5$-Alkyl)amino und Phenyl-$C_1$-$C_5$Alkylthio sind Phenylmethoxy, Phenylethoxy, Phenylpropoxy, Monobenzylamino, Monophenethylamino, Dibenzylamino, Diphenethylamino, Benzylphenethylamino, Benzylthio oder Phenethylthio. Beispiele für Carboxy-$C_1$-$C_5$Alkyl sind Carboxymethyl, Carboxyethyl, Carboxypropyl, Carboxyisopropyl, Carboxybutyl, Carboxyisobutyl, Carboxy-sek.Butyl, Carboxy-tert.Butyl, Carboxyamyl oder Carboxyisoamyl.

Halogen bei den Resten $R_1$ und $R_4$ bedeutet Fluor, Chlor, Brom, vorzugsweise Chlor.

Bei der Definition der Reste R und $R_4$ bedeutet Acyl besonders Formyl, Acetyl, Propionyl, n-Butyryl oder Benzoyl.

1-Aza-Cycloalkyl bedeutet insbesondere 1-Azacycloheptyl, 1-Azacyclohexyl oder 1-Azacyclopentyl.

Als Beispiele für Alkalimetalle seien Lithium, Natrium oder Kalium genannt. Bevorzugt ist Natrium. Beispiele für Erdalkalimetalle sind Calcium und Magnesium.

Als organischer Ammoniumrest entsprechend der Formel $(C_1\text{-}C_4Alkyl)_n(H)_mN^+$ kommt Trimethylammonium oder vorzugsweise Triethylammonium in Betracht.

Mono- oder Di-$C_1$-$C_5$Alkylamino, Mono- oder Di-$C_3$-$C_5$Alkenylamino, Mono- oder Di-(Phenyl-$C_1$-$C_5$Alkyl)amino und Mono- oder Dicycloalkylamino können mit Alkoxy, Hydroxy, Carboxy, Carboxy-$C_1$-$C_5$Alkyl oder Mono- oder Di-$C_1$-$C_5$Alkylamino substituiert sein. Phenyl-$C_1$-$C_5$Alkoxy kann mit $C_1$-$C_5$Alkyl, Halogen oder $C_1$-$C_5$Alkoxy substituiert sein. $C_1$-$C_5$Alkylthio kann durch Carboxy oder Hydroxy substituiert sein. Phenyl-$C_1$-$C_5$Alkylthio kann

durch Halogen substituiert sein. 1-Azacycloalkyl kann durch $C_1$-$C_3$Alkyl, Hydroxy oder Carboxy substituiert sein. Phenyl kann durch $C_1$-$C_3$Alkyl, Carboxy-$C_1$-$C_3$Aralkyl oder $C_1$-$C_5$Alkoxy bzw. Halogen substituiert sein. Morpholino kann durch einen oder mehrere $C_1$-$C_3$Alkylreste substituiert sein.

Wichtige Verbindungen entsprechend Formel (1) sind solche, worin

R Wasserstoff oder $C_1$-$C_3$Alkyl bedeutet.

Weitere interessante Verbindungen der Formel (1) sind solche, bei denen

$R_1$ Halogen oder einen Rest der Formel (2), (3) oder (4) bedeutet.

Im Vordergrund des Interesses stehen Verbindungen der Formel

(5)

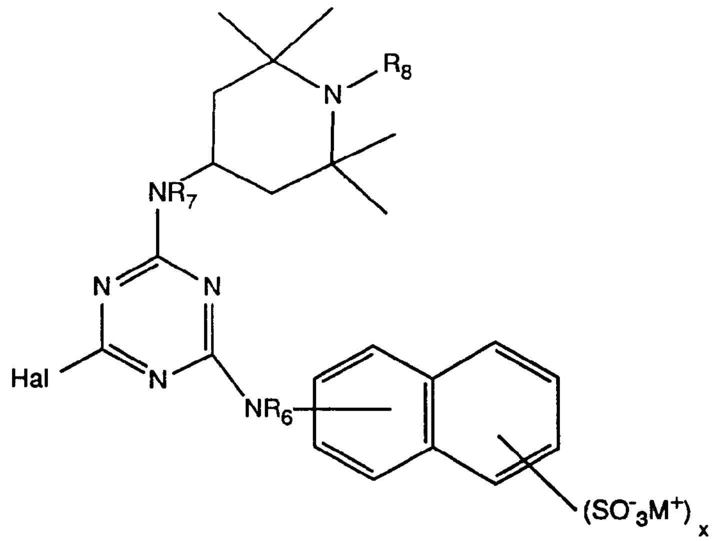

,

worin

$R_6$, $R_7$ und $R_6$ unabhängig voneinander Wasserstoff oder $C_1$-$C_3$Alkyl, Hal Halogen,

M gleich oder verschieden sein können und Wasserstoff oder Alkalimetall und

x 1 oder 2 bedeuten,

oder Verbindungen der Formel

(6)

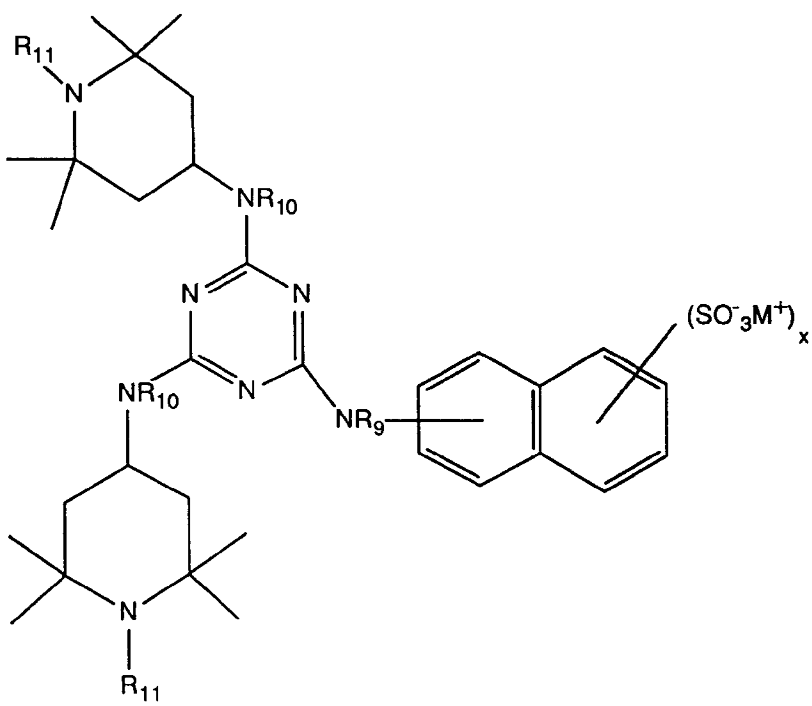

worin
$R_9$, $R_{10}$ und $R_{11}$ unabhängig voneinander Wasserstoff oder $C_1$-$C_5$Alkyl,
M gleich oder verschieden sein können und Wasserstoff oder Alkalimetall und
x 1 oder 2 bedeuten.

Weitere interessante wasserlösliche Triazinderivate entsprechen der Formel

(7)

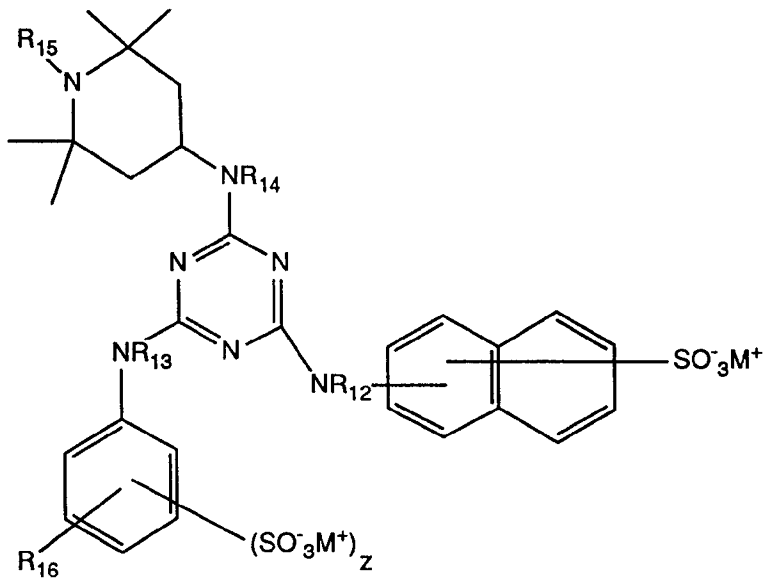

worin
$R_{12}$, $R_{13}$, $R_{14}$ und $R_{15}$ unabhängig voneinander, Wasserstoff oder $C_1$-$C_5$Alkyl,
$R_{16}$ Wasserstoff, Halogen, $C_1$-$C_5$Alkyl, $C_1$-$C_5$Alkoxy, Carboxy, Carboxy-$C_1$-$C_5$Alkyl, Mono- oder Di-$C_1$-$C_5$-Acylamino,
M gleich oder verschieden sein können und Wasserstoff oder Alkalimetall und
z 0 oder 1 bedeuten.

Weitere wichtige Vertreter der wasserlöslichen Triazinverbindungen entsprechen der Formel

(8)

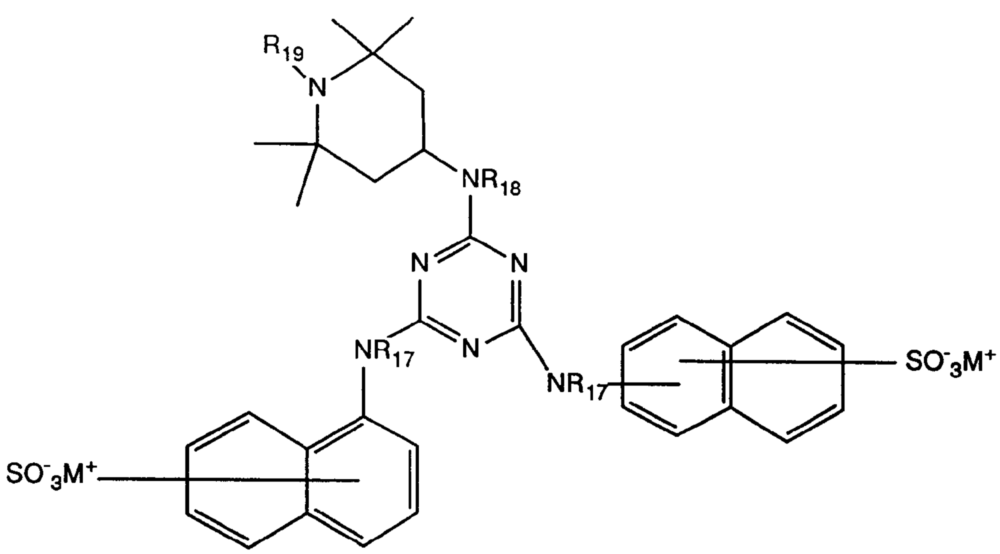

worin
$R_{17}$, $R_{18}$ und $R_{19}$ unabhängig voneinander Wasserstoff oder $C_1$-$C_5$Alkyl und
M unabhängig voneinander Wasserstoff oder Alkalimetall bedeuten.

Weitere wichtige Verbindungen entsprechend Formel (1) sind solche, worin

$R_1$ einen Rest der Formel

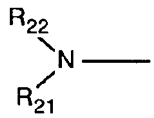

bedeutet, wobei

$R_{21}$ und $R_{22}$ unabhängig voneinander, Wasserstoff, $C_1$-$C_5$Alkyl, Cycloalkyl, unsubstituiertes oder durch $C_1$-$C_5$Alkyl substituiertes Phenyl oder

$R_1$ 1-Azacycloalkyl oder Morpholino bedeutet.

Die Herstellung der wasserlöslichen Triazinderivate entsprechend Formel (1) kann auf verschiedene Art und Weise erfolgen.

Die Herstellung der Verbindungen gemäss Formel (1) erfolgt zum Beispiel dadurch, dass man eine Verbindung der Formel

(9) 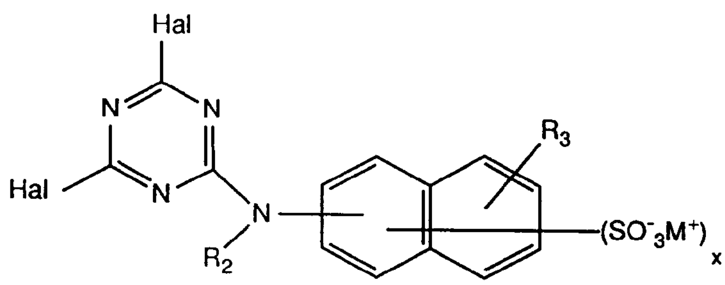

mit einem Mol der Verbindung der Formel

(4a) 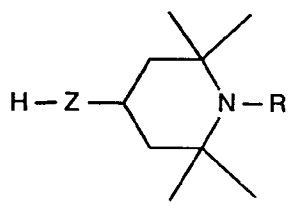 ;

oder die Verbindung der Formel (9) mit 1 Mol der Verbindung der Formel

(2a) 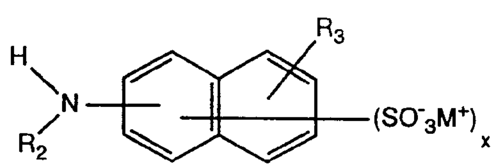

und 1 Mol der Verbindung der Formel (4a);

oder

die Verbindung der Formel (9) mit 2 Mol der Verbindung (4a) oder die Verbindung der Formel (9) mit einem Mol einer $C_1$-$C_5$Alkanolat-, $C_3$-$C_5$Alkenolat-,Cycloalkanolat-, Phenyl-$C_1$-$C_5$Alkanolat-, Phenolat-, $C_1$-$C_5$Alkylthiolat-, Cycloalkylthiolat-, Phenyl-$C_1$-$C_5$alkylthiolat- oder einer Phenylthiolatverbindung, eines Mono- oder Di-$C_1$-$C_5$Alkylamins, Mono- oder Di-$C_3$-$C_5$Alkenylamins, Mono- oder Di(phenyl-$C_1$-$C_5$Alkyl)amins, Phenylamins, Cycloalkylamins, einer 1-Azacycloalkyl-, einer Morpholinoverbindung und 1 Mol der Verbindung (4a);

oder die Verbindung der Formel (9) mit einem Mol der Verbindung der Formel

(3a) 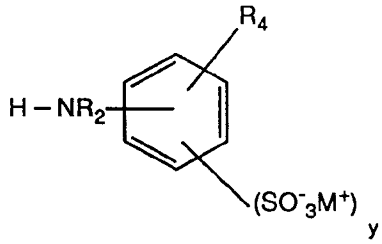

und einem Mol der Verbindung der Formel (4a) in beliebiger Reihenfolge umsetzt, wobei

R Wasserstoff; Oxyl; Hydroxy; $C_1$-$C_5$Alkyl; $C_3$-$C_5$Alkenyl; $C_1$-$C_5$Alkoxy; Acyl; oder Benzyl,

$R_2$ Wasserstoff; oder $C_1$-$C_5$Alkyl,

$R_3$ Wasserstoff; oder Hydroxy,

$R_4$ Wasserstoff; Halogen; $C_1$-$C_5$Alkyl; $C_1$-$C_5$Alkoxy; Carboxy; Carboxy-$C_1$-$C_5$Alkyl; oder Mono- oder Di-$C_1$-$C_5$-Acylamino;

M gleich oder verschieden sein können und Wasserstoff, Alkalimetall, Erdalkalimetall, Ammonium oder einen organischen Ammoniumrest der Formel $(C_1\text{-}C_4Alkyl)_n(H)_mN^+$

Z -O- oder -($NR_5$)-,

worin $R_5$ Wasserstoff oder $C_1$-$C_5$Alkyl,

Hal ein Halogenatom,

x 1 oder 2 und

y 0 oder 1 bedeutet.

Die Reihenfolge der Umsetzung der einzelnen Reaktanden mit der Verbindung der Formel (9) ist dabei beliebig.

Die Herstellung der Verbindungen der Formel (9) erfolgt durch Kondensation einer 2,4,6-Trihalogen-s-triazinverbindung mit einer Aminonaphthalinsulfonsäure. Die vorzugsweise als wässrige Dispersionen eingesetzten Triazinverbindungen sind allgemein bekannt. Besonders bevorzugt ist Cyanurchlorid.

Die Verbindungen der Formel (9) sind wertvolle Ausgangsverbindungen für die Synthese der erfindungsgemässen Verbindungen der Formeln (1), (5), (6), (7) und (8).

Verbindungen der Formel (1), worin $R_1$ $C_1$-$C_5$Alkyl oder Phenyl ist, werden hergestellt, indem man 1 Mol einer 2,4-Dihalogen-6-$C_1$-$C_5$Alkyl- bzw.
2,4-Dihalogen-6-phenyl-s-triazinverbindung nacheinander mit einem Mol der Verbindung der Formel (2a) und 1 Mol der Verbindung der Formel (4a) umsetzt.

Zur Herstellung von wasserlöslichen Triazinderivaten der Formel (5) geht man so vor, dass man 1 Mol der Verbindung der Formel (9) mit einem Mol der Piperidinverbindung der Formel (4a) umsetzt. Auf diese Weise entstehen monopiperidyl-substituierte Triazinderivate. Bei dieser Reaktionsführung liegt die Reaktionstemperatur zwischen 0 und 50, vorzugsweise 20 und 40°C, die Reaktionszeit zwischen 1 und 20, vorzugsweise 1 und 4 Stunden.

Die entsprechenden dipiperidyl-substituierten Triazinverbindungen entsprechend Formel (6) werden durch Umsetzung der Verbindung der Formel (5) mit einem Mol der Piperidinverbindung der Formel (4a) erhalten.

Dipiperidyl-substituierte Triazinderivate entsprechend Formel (6) lassen sich auch durch direkte Umsetzung von 1 Mol der Verbindung der Formel (9) mit 2 Mol der Piperidinverbindung der Formel (4a) herstellen. Bei dieser Reaktionsführung liegt die Reaktionstemperatur zwischen 20 und 100, vorzugsweise 30 und 80°C.

Die Herstellung der Verbindungen der Formel (7) erfolgt durch Umsetzung von 1 Mol der Verbindung der Formel (9) mit 1 Mol der Verbindung der Formel (3a) und 1 Mol der Verbindung der Formel (4a).

Die Herstellung von binaphthylsubstituierten Triazinderivaten entsprechend Formel (8) erfolgt dadurch, dass man ein Mol der Verbindung der Formel (9) nacheinander mit einem Mol einer Aminonaphthalinsulfonsäure der Formel (2a) und 1 Mol der Piperidinverbindung der Formel (4a) umsetzt.

Die Herstellung von wasserlöslichen Triazinderivaten der Formel (1), bei denen

$R_1$ ein Rest der Formel

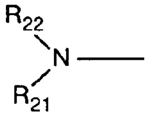

ist, worin

$R_{21}$ und $R_{22}$ unabhängig voneinander, Wasserstoff, $C_1$-$C_5$Alkyl, Cycloalkyl, unsubstituiertes oder durch $C_1$-$C_5$Alkyl substituiertes Phenyl oder

$R_1$ 1-Azacycloalkyl oder Morpholino bedeutet,

erfolgt dadurch, dass man eine Verbindung der Formel (9) mit einer Piperidinverbindung der Formel (4a) und der entsprechenden N-Alkylverbindung oder Aminophenylverbindung der Formel

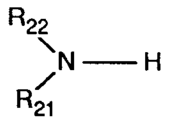

umsetzt. Die Reihenfolge der Umsetzungen mit der Piperidinverbindung der Formel (4a) und der N-Alkylverbindung hängt von der Reaktivität der jeweiligen Verbindungen ab. In der Regel geht man so vor, dass zunächst mit der Verbindung umgesetzt wird, die die geringere Reaktivität aufweist.

Die bei den Kondensationsreaktionen entstehende Halogenwasserstoffsäure kann durch das Endprodukt selbst oder durch Hinzufügen einer weiteren Base, wie beispielsweise wässrigem Ammoniak, Alkalimetallhydroxiden, Alkalimetallcarbonaten, -hydrogencarbonaten oder einer organischen Base, wie beispielsweise Triethylamin, abgefangen werden.

Die Verbindungen der Formeln (1) und (5) bis (8) werden vorzugsweise als Natriumsalze eingesetzt. Dazu werden sie beispielsweise mit der äquivalenten Menge Natronlauge gelöst.

Die neuen Triazinderivate eignen sich dazu, die photochemische und thermische Stabilität von ungefärbten und gefärbten Polyamid-Fasermaterialien zu erhöhen. Einen weiteren Erfindungsgegenstand bildet daher ein Verfahren zur photochemischen und thermischen Stabilisierung von Polyamidfasermaterialien. Dieses Verfahren ist dadurch gekennzeichnet, dass man gefärbte oder ungefärbte Polyamid-Fasermaterialien mit einem wasserlöslichen Triazinderivat der Formel

(1)

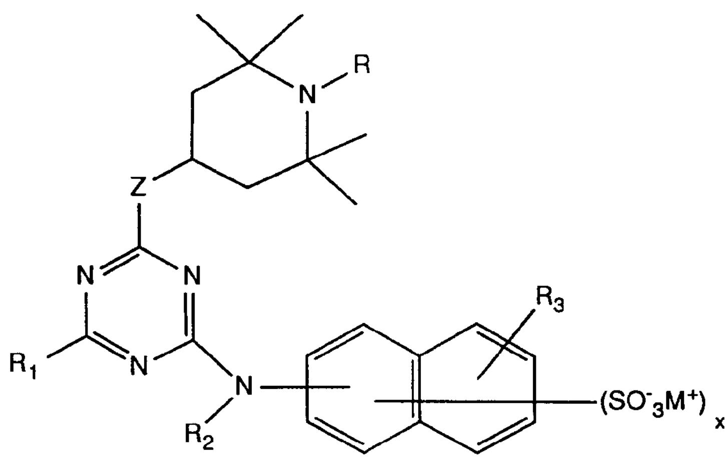

worin

R Wasserstoff; Oxyl; Hydroxy; $C_1$-$C_5$Alkyl; $C_3$-$C_5$Alkenyl; $C_1$-$C_5$Alkoxy; Acyl; oder Benzyl

$R_1$ Halogen; $C_1$-$C_5$Alkyl; Amino; $C_1$-$C_5$Alkoxy; $C_3$-$C_5$Alkenyloxy; Cycloalkoxy; nicht substituiertes oder im Phenylteil durch Halogen, $C_1$-$C_5$Alkyl, $C_1$-$C_5$Alkoxy, Carboxy, Carboxy-$C_1$-$C_5$Alkyl, Carbamoyl, Mono- oder Di-$C_1$-$C_5$-Acylamino oder Acyl substituiertes Phenoxy; Phenyl; Phenyl-$C_1$-$C_5$Alkyl; Phenylthio; Phenyl-$C_1$-$C_5$Alkylthio; Mono- oder Diphenyl-$C_1$-$C_5$Alkylamino; $C_1$-$C_5$Alkylthio; Cycloalkylthio; nicht substituiertes oder durch Hydroxy oder Carboxy substituiertes Mono- oder Di-$C_1$-$C_5$Alkylamino, wobei die Alkylkette durch ein Sauerstoffatom unterbrochen sein kann; Mono- oder Di-$C_3$-$C_5$Alkenylamino; nicht substituiertes oder durch $C_1$-$C_5$Alkyl substituiertes Mono- oder Dicycloalkylamino; nicht substituiertes oder durch $C_1$-$C_5$Alkyl, Hydroxy oder Carboxy substituiertes 1-Azacycloalkyl; nicht substituiertes oder durch $C_1$-$C_5$Alkyl substituiertes Morpholino; einen Rest der Formel

(2) 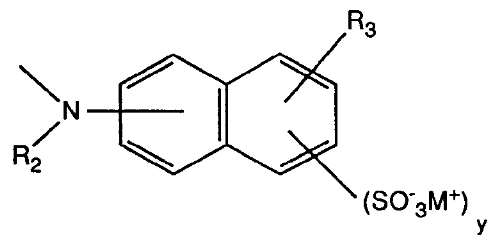 ,

einen Rest der Formel

(3) 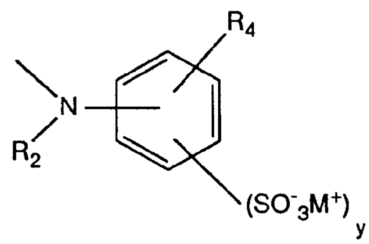

oder einen Rest der Formel

(4) 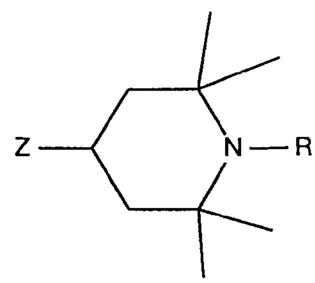

$R_2$ Wasserstoff oder $C_1$-$C_5$Alkyl,
$R_3$ Wasserstoff oder Hydroxy,
$R_4$ Wasserstoff; Halogen; $C_1$-$C_5$Alkyl; $C_1$-$C_5$Alkoxy; Carboxy; Carboxy-$C_1$-$C_5$Alkyl; Acyl; Carbamoyl; oder Mono- oder Di-$C_1$-$C_5$-Acylamino,
M gleich oder verschieden sein können und Wasserstoff; Alkalimetall; Erdalkalimetall; Ammonium; oder einen organischen Ammoniumrest der Formel $(C_1\text{-}C_4Alkyl)_n(H)_mN^+$; und
Z -O-; oder -($NR_5$)-,
$R_5$ Wasserstoff; oder $C_1$-$C_5$Alkyl,
m 0 bis 3;
n 1 bis 4; und die Summe von m+n = 4;
x 1 oder 2;
y 0 oder 1bedeuten;

und, wenn $R_1$ einen Rest der Formel (2) oder (3) bedeutet und y 1 ist, x in Formel (1) 1 ist, behandelt.

Die neuartigen Verbindungen stellen Vertreter der sterisch gehinderten Amine ("HALS"-Stabilisatoren) dar und können aus üblichen Flotten nach gängigen Methoden auf die Polyamid-Fasermaterialien aufgebracht werden.

Diese Verbindungen werden erfindungsgemäss aus wässrigem Bad appliziert, das die Verbindungen in einer Menge von 0,005 bis 10 Gew.-%, vorzugsweise 0,05 bis 2 Gew.-% enthält. Vorzugsweise werden die Verbindungen dem Färbebad zugefügt. Die Applikation kann dabei vor oder nach dem Färben oder während des Färbens, nach einem Auszieh-oder Kontinueverfahren erfolgen. Die Applikation während des Färbens ist bevorzugt.

Beim Ausziehverfahren kann das Flottenverhältnis innerhalb eines weiten Bereiches gewählt werden, z.B. 1:5 bis 1:300, vorzugsweise 1:10 bis 1:50. Man arbeitet zweckmässig bei einer Temperatur von 30 bis 120°C, vorzugsweise 50 bis 98°C.

Beim Kontinueverfahren beträgt der Flottenauftrag zweckmässig 30-400 Gew.-%, vorzugsweise 75-250 Gew.-%. Zur Fixierung der applizierten Farbstoffe und der neuen Verbindungen wird das Fasermaterial einer Hitzebehandlung unterworfen. Der Fixierprozess kann auch nach der Kaltverweilmethode erfolgen.

Die Hitzebehandlung erfolgt vorzugsweise durch ein Dämpfverfahren unter Behandlung in einem Dämpfer mit gegebenenfalls überhitztem Dampf bei einer Temperatur von 98 bis 105°C während z.B. 1 bis 7, vorzugsweise 1 bis 5 Minuten. Die Fixierung der Farbstoffe und der erfindungsgemässen Verbindungen gemäss dem Kaltverweilverfahren kann durch Lagerung der imprägnierten und vorzugsweise aufgerollten Ware bei Raumtemperatur (15 bis 30°C), z.B. während 3 bis 24 Stunden erfolgen, wobei die Kaltverweilzeit bekanntlich von der Art des applizierten Farbstoffes abhängig ist.

Nach Beendigung des Färbeprozesses bzw. der Fixierung werden die hergestellten Färbungen auf übliche Weise gespült und getrocknet.

Man erhält mit dem erfindungsgemässen Verfahren Polyamid-Färbungen und -fasern mit guter photochemischer und thermischer Stabilität.

Die Färbungen, die erfindungsgemäss photochemisch und thermisch stabilisiert werden, sind solche, die mit Säure- oder Metallkomplexfarbstoffen, z.B. 1:2-Chrom-, 1:2-Kobalt- oder Kupfer-Komplexfarbstoffen, aber auch Dispersions- und Reaktivfarbstoffen hergestellt werden.

Beispiele für solche Farbstoffe sind in Colour Index, 3. Auflage, 1971, Band 4, beschrieben.

Unter Polyamidfasermaterial wird synthetisches Polyamid, wie z.B. Polyamid 6, Polyamid 6,6 oder Polyamid 12, sowie modifiziertes Polyamid, z.B. basisch anfärbbares Polyamid verstanden. Neben den reinen Polyamidfasern kommen vor allem auch Fasermischungen aus Polyurethan und Polyamid in Betracht, so z.B. Trikotmaterial aus Polyamid/Polyurethan im Mischungsverhältnis 70:30. Grundsätzlich kann das reine oder gemischte Polyamidfasermaterial in den verschiedensten Verarbeitungsformen vorliegen, wie z.B. als Faser, Garn, Gewebe, Gewirke, Vlies oder Flormaterial.

Die neuen wasserlöslichen Verbindungen eignen sich besonders vorteilhaft zur Behandlung von Polyamidfasermaterial, das Licht und Hitze ausgesetzt wird und z.B. als Autopolsterstoff oder Teppich Verwendung findet.

Die folgenden Beispiele veranschaulichen die Erfindung. Teile bedeuten Gewichtsteile und Prozente Gewichtsprozente.

Herstellungsbeispiele:

Beispiel 1:

Eine neutrale Suspension von 19,7 g 1-N-(2,4-Dichlor-6-s-triazinyl)aminonaphthalin-6-sulfonsäure Natriumsalz in 100 ml dest. Wasser wird unter schnellem Rühren mit 7,8 g 4-Amino-2,2,6,6-tetramethylpiperidin versetzt. Anschliessend rührt man während 2 Stunden bei 35°C nach. Nach dem Abkühlen auf Raumtemperatur wird das Reaktionsgemisch abfiltriert, mit dest. Wasser gewaschen und bei 50°C im Vakuum getrocknet. Man erhält 11,5 g eines weissen Pulvers der Formel

(101) 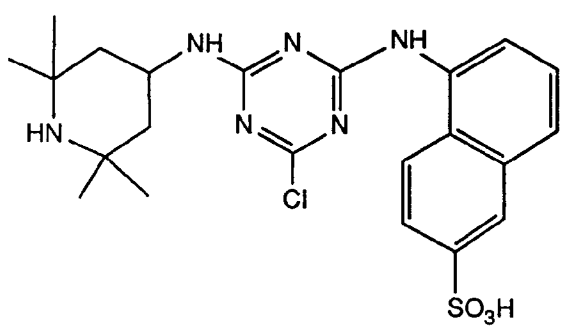 .

Die Verbindung zeigt das langwelligste Absorptionsmaximum bei 280 nm (Wasser).

Beispiel 2:

Zu einer neutralen Suspension von 16,4 g eines Monokondensationsproduktes aus Cyanurchlorid und 2-Aminonaphthalin-6-sulfonsäure Natriumsalz (Aktivgehalt 60%) in 100 ml dest. Wasser werden unter Rühren bei Raumtemperatur 2,3 g Anilin innerhalb von 5 Minuten zugetropft. Durch Zutropfen von konzentrierter Natronlauge hält man den pH-Wert des Reaktionsgemisches zwischen 6 und 7. Nach einer Rührzeit von 2 Stunden ist die Kondensation beendet. Man verdünnt mit 50 ml dest. Wasser und versetzt rasch mit 3,9 g 4-Amino-2,2,6,6-tetramethylpiperidin. Anschliessend rührt man während 16 Stunden bei 80°C. Nach dem Abkühlen auf Raumtemperatur wird der entstandene Niederschlag abgesaugt, mit destilliertem Wasser gewaschen und im Vakuum bei 65°C getrocknet. Man erhält 13,8 g einer farblosen Verbindung der Formel

(102)

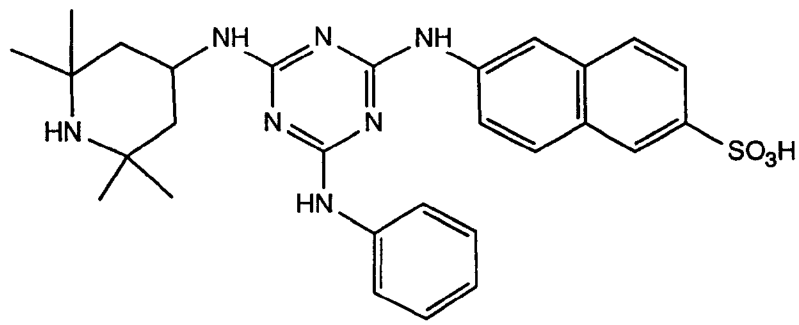

mit dem langwelligsten Absorptionsmaximum bei 299 nm (Boraxpuffer).

Beispiel 3:

Zu einer neutralen Suspension von 14,8 g eines Monokondensationsproduktes aus Cyanurchlorid und 3-Aminonaphthalin-1,5-disulfonsäure Dinatriumsalz (Aktivgehalt 67%) in 100 ml dest. Wasser werden bei Raumtemperatur 1,9 g Anilin hinzugegeben. Unter Rühren erwärmt man auf 40°C und hält durch Zutropfen von konzentrierter Natronlauge den pH-Wert des Reaktionsgemisches zwischen 6 und 7. Nach beendeter Kondensation (1 Stunde) trägt man 3,1 g 4-Amino-2,2,6,6-tetramethylpiperidin ein und erhitzt während 16 Stunden auf 75°C. Man kühlt auf Raumtemperatur ab und salzt mit 12 g Kochsalz aus. Der entstandene Niederschlag wird abgesaugt, mit 10%iger Kochsalzlösung gewaschen und im Vakuum bei 65°C getrocknet. Man erhält 18,2 g eines Pulvers der Formel

(103)

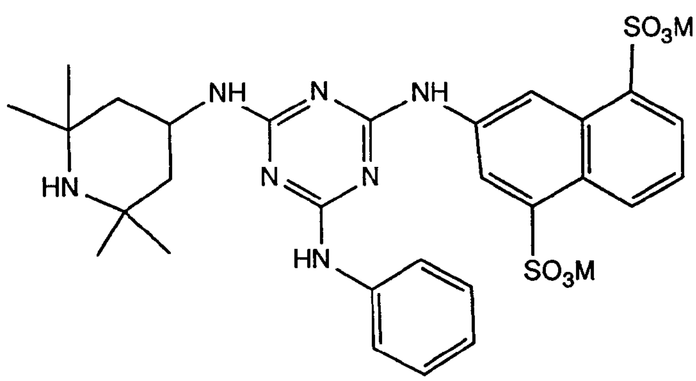

M = H, Na

Das langwelligste Absorptionsmaximum beträgt 337 nm (Wasser).

Beispiel 4:

Eine Lösung von 3,7 g Cyanurchlorid in 20 ml Aceton wird auf 100 ml Eiswasser ausgetragen und anschliessend mit 4,5 g 1-Aminonaphthalin-6-sulfonsäure versetzt. Man rührt bei 0 bis 5°C und hält den pH-Wert des Gemisches durch Zutropfen von konz. Natronlauge zwischen 2,5 und 3. Nach einer Stunde wird auf Raumtemperatur erwärmt und nochmals 4,5 g 1-Aminonaphthalin-6-sulfonsäure eingetragen. Man hält den pH-Wert durch weiteres Zutropfen von konzentrierter Natronlauge zwischen 6 und 7 und rührt während 2 Stunden aus. Zuletzt gibt man rasch 3,75 g 4-Amino-2,2,6,6-tetramethylpiperidin zum Reaktionsgemisch und erhitzt während 16 Stunden auf 70°C. Der entstandene Niederschlag wird bei Raumtemperatur abgesaugt, mit Aceton und Wasser gewaschen und im Vakuum bei 50°C getrocknet. Man erhält 10,3 g eines weissen Pulvers der Formel

(104)

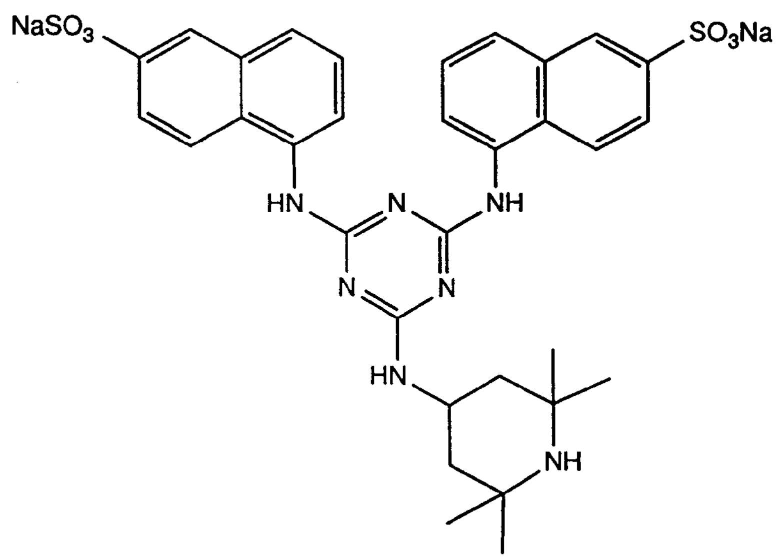

.

Die Verbindung zeigt das langwelligste Absorptionsmaximum bei 292 nm (Boraxpuffer).

Beispiel 5:

Zu einer neutralen Suspension von 13,1 g eines Monokondensationsproduktes aus Cyanurchlorid und 2-Aminonaphthalin-6-sulfonsäure Natriumsalz (Aktivgehalt 60%) in 100 ml dest. Wasser werden bei Raumtemperatur rasch 9,4 g 4-Amino-2,2,6,6-tetramethylpiperidin eingetragen und das Gemisch während 18 Stunden bei 80°C gerührt. Man filtriert nach dem Abkühlen auf Raumtemperatur, wäscht mit Wasser und trocknet im Vakuum bei 65°C. Man erhält 13,3 g eines farblosen Pulvers der Formel

(105)

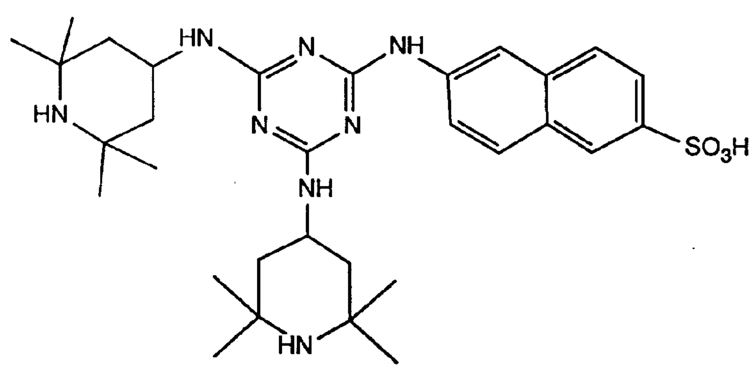

Das langwelligste Absorptionsmaximum beträgt 300 nm (Boraxpuffer).

Beispiel 6:

Zu einer neutralen Suspension von 14,8 g eines Monokondensationsproduktes aus Cyanurchlorid und 3-Aminonaphthalin-1,5-disulfonsäure Dinatriumsalz (Aktivgehalt 67%) in 100 ml dest. Wasser werden bei Raumtemperatur 6,25 g 4-Amino-2,2,6,6-tetramethylpiperidin eingetragen und das Gemisch während 48 Stunden bei 75°C gerührt. Man filtriert nach dem Abkühlen auf Raumtemperatur, wäscht mit Wasser und trocknet im Vakuum bei 65°C. Man erhält 13,6 g eines weissen Pulvers der Formel

(106)

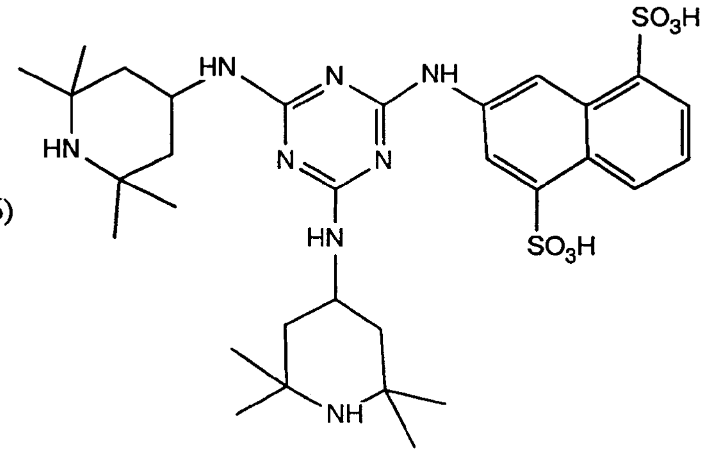

Das langwelligste Absorptionsmaximum beträgt 337 nm (Wasser).

Beispiel 7:

Eine Lösung von 6,3 g 2-Phenyl-4,6-dichlor-s-triazin in 15 ml Aceton wird auf 50 ml dest. Wasser ausgetragen. Anschliessend werden 6,9 g 2-Aminonaphthalin-6-sulfonsäure Natriumsalz unter raschem Rühren eingetragen. Man erhöht die Temperatur auf 40°C und hält den pH-Wert des Reaktionsgemisches durch Zutropfen von konz. Natronlauge bei 6. Nach 4 Stunden ist die Kondensation beendet. Danach gibt man rasch 4,4 g 4-Amino-2,2,6,6-tetramethylpiperidin zum Gemisch und erhitzt während 16 Stunden auf 75°C. Nach dem Abkühlen auf Raumtemperatur saugt man ab, wäscht mit Wasser und trocknet im Vakuum bei 65°C. Man erhält 11,9 g eines farblosen Pulvers der Formel

(107)

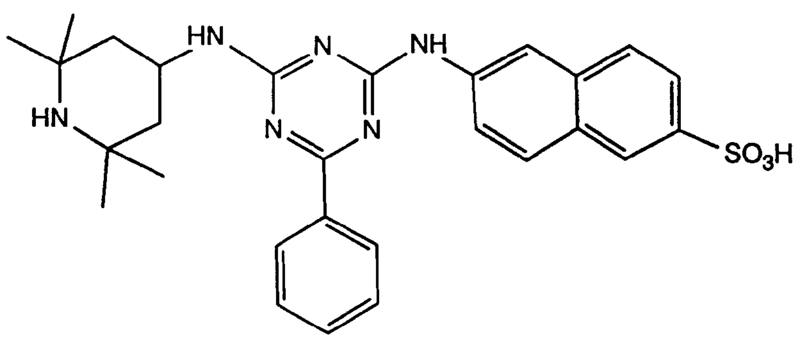

Das langwelligste Absorptionsmaximum beträgt 304 nm (Boraxpuffer).

Beispiel 8:

Eine Suspension von 5,4 g 2-Phenyl-4,6-dichlor-s-triazin in 15 ml Aceton/50 ml dest. Wasser wird unter raschem Rühren mit einer Lösung von 8,3 g 3-Aminonaphthalin-1,5-disulfonsäure Dinatriumsalz in 50 ml dest. Wasser versetzt. Man erwärmt das Reaktionsgemisch während 4 Stunden auf 35°C und hält den pH-Wert des Reaktionsgemisches durch Zutropfen von konz. Natronlauge zwischen 5 und 6. Danach gibt man rasch 3,75 g 4-Amino-2,2,6,6-tetramethylpiperidin zum Gemisch und rührt während 16 Stunden bei 75°C nach. Nach dem Abkühlen auf Raumtemperatur wird filtriert, mit wenig Wasser und Aceton gewaschen und im Vakuum bei 65°C getrocknet. Man erhält 12,3 g eines Pulvers der Formel

(108) 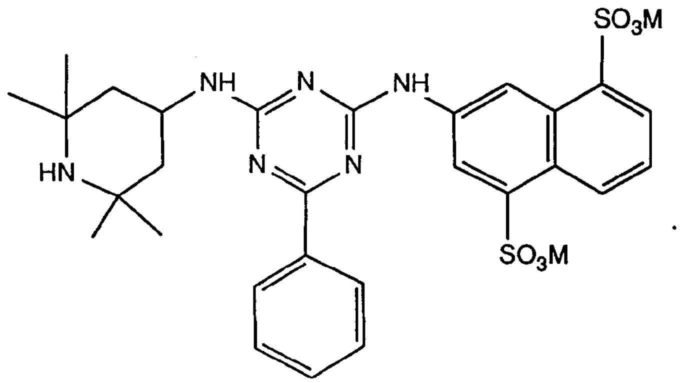

M = Na, H

Die langwelligste Absorptionsbande dieser Verbindung wird als Schulter bei 340 nm (Wasser) beobachtet.

Beispiel 9:

Zu einer neutralen Suspension von 16,4 g eines Monokondensationsproduktes aus Cyanurchlorid und 2-Aminonaphthalin-6-sulfonsäure Natriumsalz (Aktivgehalt 60%) in 100 ml dest. Wasser werden bei Raumtemperatur unter Rühren 2,18 g Morpholin innerhalb von 5 Minuten zugetropft. Durch Zutropfen von konz. Natronlauge hält man den pH-Wert des Reaktionsgemisches zwischen 6 und 7. Man rührt während 2 Stunden bei Raumtemperatur und zuletzt noch während 1,5 Stunden bei 40°C. Danach wird das Reaktionsgemisch rasch mit 3,9 g 4-Amino-2,2,6,6-tetramethylpiperidin versetzt. Man erhöht die Temperatur auf 80°C und rührt während 16 Stunden nach. Nach dem Abkühlen auf Raumtemperatur wird der Niederschlag abgesaugt, mit Wasser gewaschen und im Vakuum bei 65°C getrocknet. Man erhält 11,9 g einer farblosen Verbindung der Formel

(109) 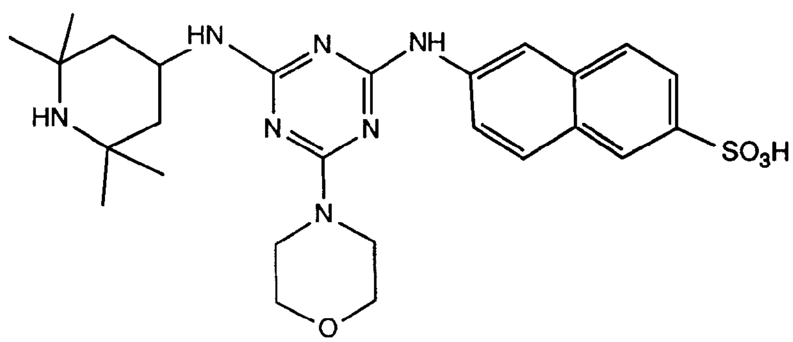

Das langwelligste Absorptionsmaximum beträgt 300 nm (Boraxpuffer).

Beispiel 10:

Verfährt man wie in Beispiel 9 beschrieben, setzt jedoch anstelle von 2,18 g Morpholin 2,76 g Thiophenol ein, so erhält man 11,9 g eines farblosen Produktes der Formel

(110)

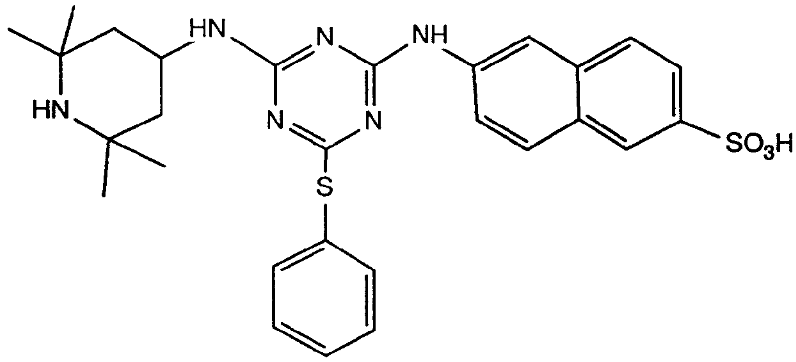

mit dem langwelligsten Absorptionsmaximum bei 304 nm (Boraxpuffer).

Beispiel 11:

Zu einer neutralen Suspension von 15,8 g eines Monokondensationsproduktes (Aktivgehalt 60%) von Cyanurchlorid und 2-Aminonaphthalin-6-sulfonsäure in 100 ml dest. Wasser werden rasch 3,77 g 4-Amino-2,2,6,6-tetramethylpiperidin eingetragen und das Gemisch während 2 Stunden auf 40°C erwärmt. Nach dem Abkühlen auf Raumtemperatur wird der Niederschlag abfiltriert, und mit dest. Wasser nachgewaschen. Nach dem Trocknen bei 40°C im Vakuum erhält man 12,35 g eines weissen Pulvers der Formel

(111)

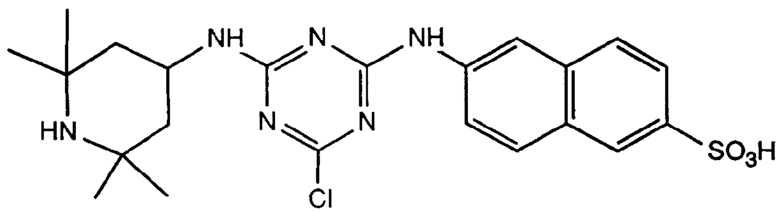

.

Das langwelligste Absorptionsmaximum beträgt 300 nm (Boraxpuffer).

Beispiel 12:

Eine neutrale Suspension von 11,05 g eines Monokondensationsproduktes (Aktivgehalt 67%) von Cyanurchlorid mit 3-Aminonaphthalin-1,5- disulfonsäure in 100 ml dest. Wasser wird bei Raumtemperatur rasch mit 2,34 g 4-Amino-2,2,6,6-tetramethylpiperidin versetzt. Man erhitzt die Reaktionslösung anschliessend während einer Stunde auf 40°C. Nach dem Abkühlen auf Raumtemperatur giesst man das Gemisch auf 500 ml Aceton und saugt den Niederschlag ab. Man wäscht mit Aceton nach und trocknet bei 40°C im Vakuum. Man erhält man 10,2 g eines kristallinen Produktes der Formel

(112)

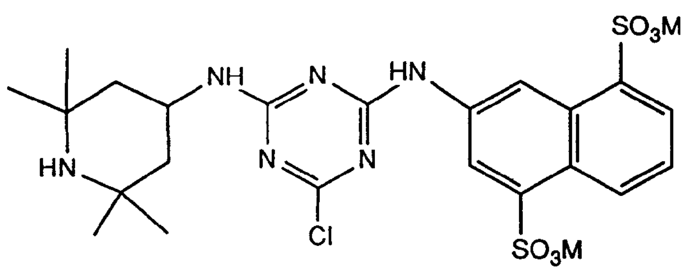

M = H, Na

Die Verbindung weist bei 335 nm (Schulter) in Wasser das langwelligste Absorptionsmaximum auf.

Beispiel 13:

Verfährt man wie in Beispiel 3 beschrieben, setzt aber anstelle von 1,9 g Anilin 1,74 g Morpholin ein, so erhält man eine Verbindung der Formel

(113) 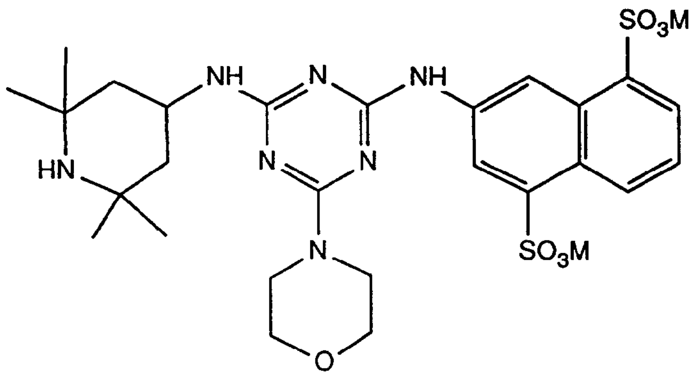

M = H, Na

Das langwelligste Absorptionsmaximum beträgt 340 nm (Wasser).

Beispiel 14:

Verfährt man wie in Beispiel 3 beschrieben, setzt aber anstelle von 1,9 g Anilin 2,2 g Thiophenol ein, so wird eine Verbindung der Formel

(114) 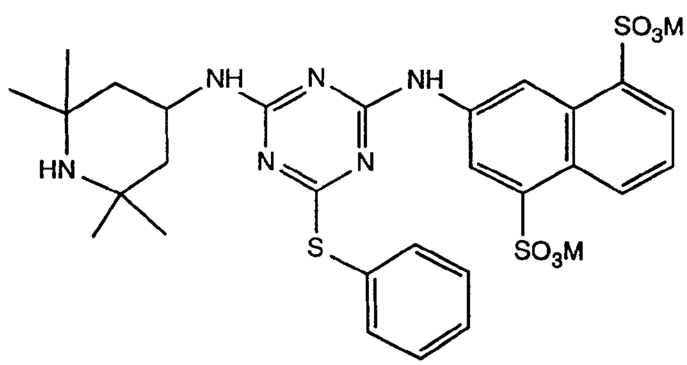

M = H, Na

erhalten, welche bei 340 nm (Schulter) in Wasser das langwelligste Absorptionsmaximum aufweist.

Applikationsbeispiele:

Beispiel 15:

Es werden 14 Muster einer Polyamid 6-Wirkware von je 10 g vorbereitet und in einem Laborfärbeapparat, z.B. einem ®AHIBA-Färbeapparat, bei einem Flottenverhältnis von 1:25 "blindgefärbt" (d.h. ohne Farbstoff behandelt: Flotten 1, 3, 5, 7, 9, 11 und 13) und gefärbt (Flotten 2, 4, 6, 8, 10, 12 und 14).

Es werden somit 14 Flotten bereitet, die jeweils 0,5 g/l Mononatriumphosphat, 1,5 g/l Dinatriumphosphat (≙ pH-Wert 7) und ein Färbereihilfsmittel (®Albegal SW) enthalten. In den Färbeflotten 2, 4, 6, 8, 10, 12 und 14 werden folgende Farbstoffe in gelöster Form zugesetzt:

0,04 % des Farbstoffgemisches bestehend aus 81 Teilen der Verbindung der Formel

(Ia)

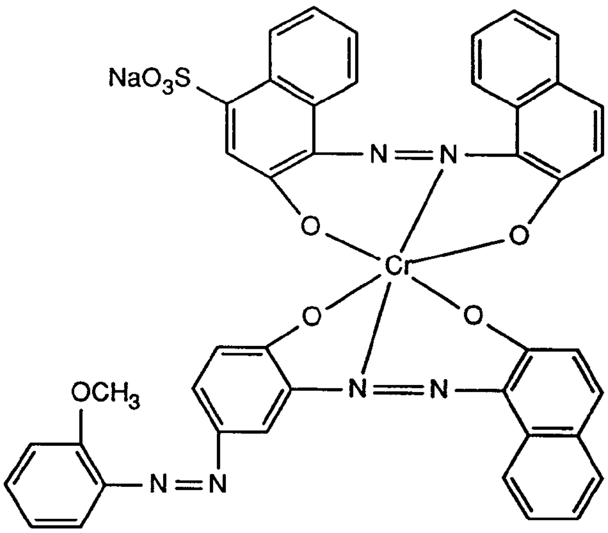

1:2-Cr-Komplex

und 12 Teilen der Verbindung der Formel

(Ib)

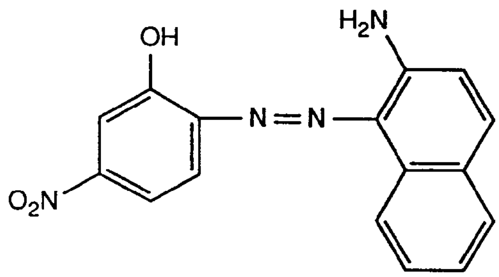

1:2-Co-Komplex

(Rest von 7 Teilen sind Salze und Tenside)

und 0,002 % des Farbstoffes der Formel

(II)

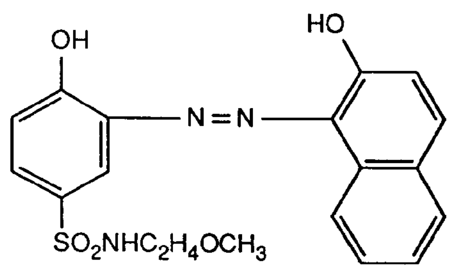

1:2-Co-Komplex

Die Flotten 3 und 4 enthalten zusätzlich noch 0,1 % des Natriumsalzes der Verbindung der Formel (101).
Die Flotten 5 und 6 enthalten je 0,1% des Natriumsalzes der Verbindung der Formel (102).
Die Flotten 7 und 8 enthalten je 0,1% des Natriumsalzes der Verbindung der Formel (104).
Die Flotten 9 und 10 enthalten je 0,1% des Natriumsalzes der Verbindung der Formel (105).
Die Flotten 11 und 12 enthalten je 0,1 % des Natriumsalzes der Verbindung der Formel (110).
Die Flotten 13 und 14 enthalten je 0,1 % des Natriumsalzes der Verbindung der Formel (109).

Mit dem vorbereiteten Textilmaterial geht man bei 40°C in die Flotten ein, verweilt 10 Minuten bei dieser Temperatur und erhitzt mit 2°/Minute auf 95°C. Nach einer Behandlungszeit von 20 Minuten gibt man 2 % Essigsäure (80%ig) hinzu und behandelt weitere 20 Minuten. Schliesslich kühlt man auf 60°C ab, spült, zentrifugiert und trocknet die Muster.

Die behandelten Muster werden nach DIN 75.202 (=FAKRA) während 216 Stunden belichtet. Reissfestigkeit und Dehnung werden nach SN 198.461 ermittelt. Die Lichtechtheiten der Färbungen werden ebenfalls nach DIN 75 202 ermittelt.

Die Ergebnisse sind in der nachfolgenden Tabelle I aufgeführt:

Tabelle I

| Flotte Nr./ Zugesetzte Verbindung | Reissfestigkeit/ Dehnung [%] nach Belichtung 216 h FAKRA | Lichtechtheit nach FAKRA 144 H [3] |
|---|---|---|
| Flotte 1: kein Zusatz | 2,7/11,9 | - [1] |
| Flotte 2: kein Zusatz | - [1] | << 1H [2] |
| Flotte 3: +Verbindung (101) | 51,9/48,2 | - [1] |
| Flotte 4 +Verbindung (101) | - [1] | 1-2 |
| Flotte 5: +Verbindung (102) | 59,0/53,9 | - [1] |
| Flotte 6: +Verbindung (102) | - [1] | 2-3 |
| Flotte 7: +Verbindung (104) | 53,5/52,1 | - [1] |
| Flotte 8: +Verbindung (104) | - [1] | 2 |
| Flotte 9: +Verbindung (105) | 40,4/40,9 | - [1] |
| Flotte 10: +Verbindung (105) | - [1] | 2 |
| Flotte 11: +Verbindung (110) | 54,5/50,4 | - [1] |
| Flotte 12: +Verbindung (110) | - [1] | 2-3 |
| Flotte 13: +Verbindung (109) | 47,4/46,2 | - [1] |
| Flotte 14: +Verbindung (109) | - [1] | 2-3 |

[1] nicht geprüft

[2] Muster nicht mehr reissfest; ausgebleicht

[3] entspricht 2 Belichtungscyclen

Die Ergebnisse in der Tabelle I zeigen, dass die erfindungsgemässen Verbindungen eine deutliche Verbesserung der photochemischen Stabilität der Polyamid-Fasern und -färbungen bewirken.

## Patentansprüche

1. Wasserlösliche Verbindungen der Formel

(1) 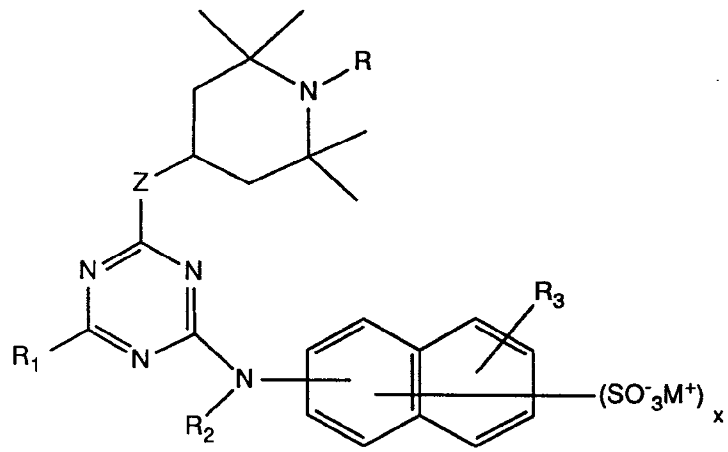

worin

R Wasserstoff; Oxyl; Hydroxy; $C_1$-$C_5$Alkyl; $C_3$-$C_5$Alkenyl; $C_1$-$C_5$Alkoxy; Acyl; oder Benzyl

$R_1$ Halogen; $C_1$-$C_5$Alkyl; Amino; $C_1$-$C_5$Alkoxy; $C_3$-$C_5$Alkenyloxy; Cycloalkoxy; nicht substituiertes oder im Phenylteil durch Halogen, $C_1$-$C_5$Alkyl, $C_1$-$C_5$Alkoxy, Carboxy, Carboxy-$C_1$-$C_5$Alkyl, Carbamoyl, Mono- oder Di-$C_1$-$C_5$-Acylamino oder Acyl substituiertes Phenoxy; Phenyl; Phenyl-$C_1$-$C_5$Alkyl; Phenylthio;
Phenyl-$C_1$-$C_5$Alkylthio; Mono- oder Diphenyl-$C_1$-$C_5$Alkylamino; $C_1$-$C_5$Alkylthio; Cycloalkylthio; nicht substituiertes oder durch Hydroxy oder Carboxy substituiertes Mono- oder Di-$C_1$-$C_5$Alkylamino, wobei die Alkylkette durch ein Sauerstoffatom unterbrochen sein kann; Mono- oder Di-$C_3$-$C_5$Alkenylamino; nicht substituiertes oder durch $C_1$-$C_5$Alkyl substituiertes Mono- oder Dicycloalkylamino; nicht substituiertes oder durch $C_1$-$C_5$Alkyl, Hydroxy oder Carboxy substituiertes 1-Azacycloalkyl; nicht substituiertes oder durch $C_1$-$C_5$Alkyl substituiertes Morpholino; einen Rest der Formel

(2) 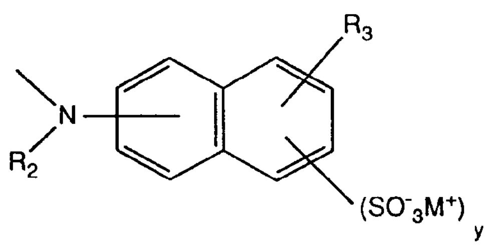 ,

einen Rest der Formel

(3) 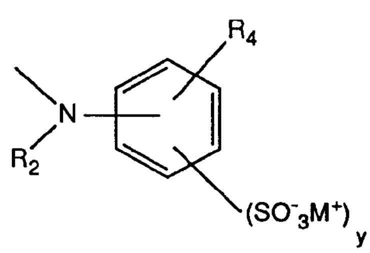

oder einen Rest der Formel

(4) 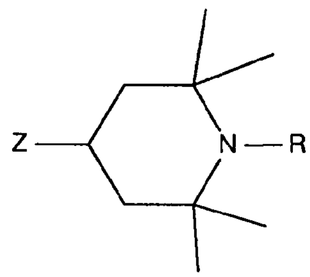

| | |
|---|---|
| $R_2$ | Wasserstoff; oder $C_1$-$C_5$Alkyl, |
| $R_3$ | Wasserstoff; oder Hydroxy, |
| $R_4$ | Wasserstoff; Halogen; $C_1$-$C_5$Alkyl; $C_1$-$C_5$Alkoxy; Carboxy; Carboxy-$C_1$-$C_5$Alkyl; Acyl; Carbamoyl; oder Mono- oder Di-$C_1$-$C_5$-Acylamino, |
| M | gleich oder verschieden sein können und Wasserstoff; Alkalimetall; Erdalkalimetall; Ammonium; oder einen organischen Ammoniumrest der Formel $(C_1\text{-}C_4Alkyl)_n(H)_mN^+$; und |
| Z | -O-; oder -($NR_5$)-, |
| $R_5$ | Wasserstoff; oder $C_1$-$C_5$Alkyl, |
| m | 0 bis 3; |
| n | 1 bis 4; und die Summe m + n = 4 |
| x | 1 oder 2 und |
| y | 0 oder 1,bedeuten |

und, wenn $R_1$ einen Rest der Formel (2) oder (3) bedeutet und y 1 ist, x in Formel (1) 1 ist.

2. Verbindungen gemäss Anspruch 1, worin
R Wasserstoff oder $C_1$-$C_5$Alkyl bedeutet.

3. Verbindungen gemäss Anspruch 1 oder 2, worin
$R_1$ Halogen, einen Rest der Formel (2), (3) oder (4) bedeutet.

4. Verbindungen gemäss einem der Ansprüche 1 bis 3 der Formel

(5) 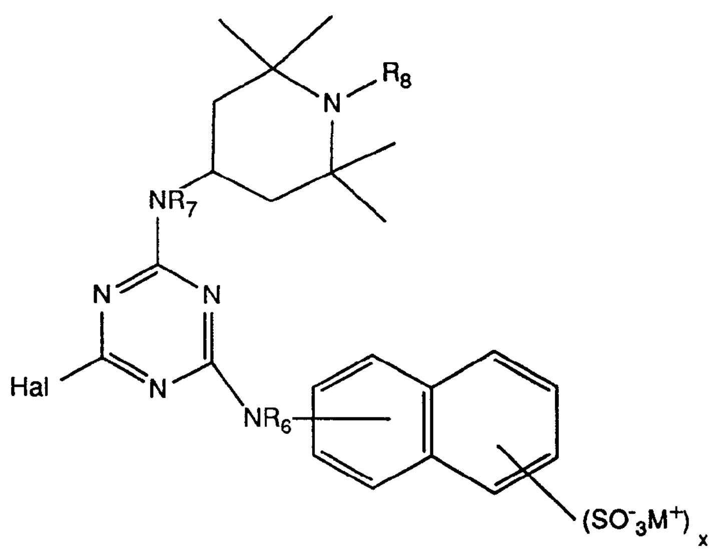 ,

worin
$R_6$, $R_7$ und $R_5$ unabhängig voneinander Wasserstoff oder $C_1$-$C_5$Alkyl, Hal Halogen
M gleich oder verschieden sein können und Wasserstoff oder Alkalimetall und
x 1 oder 2 bedeuten.

5. Verbindungen gemäss Anspruch 1 oder 2 der Formel

(6)

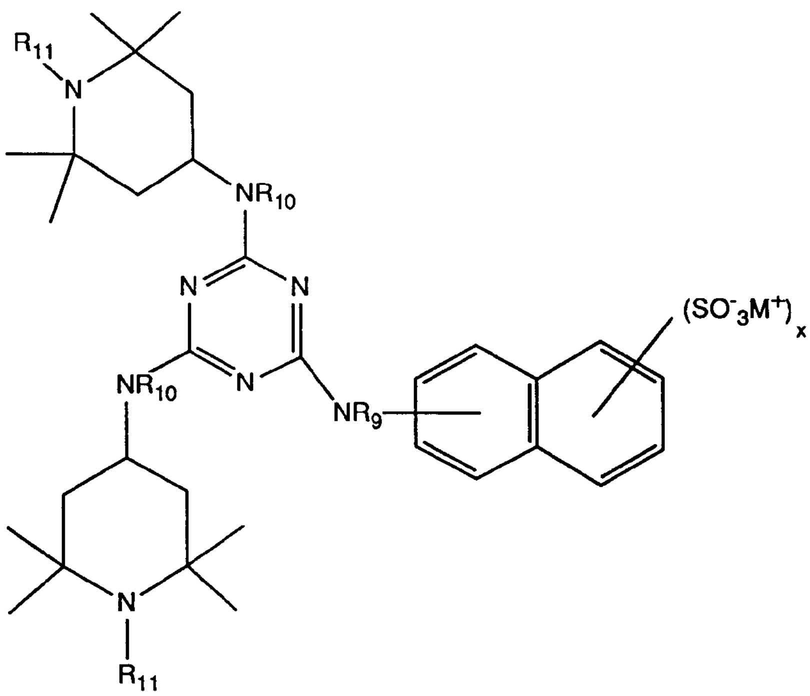

worin
$R_9$, $R_{10}$ und $R_{11}$ unabhängig voneinander Wasserstoff oder $C_1$-$C_5$Alkyl,
M gleich oder verschieden sein können und Wasserstoff oder Alkalimetall und
x 1 oder 2 bedeuten.

6. Verbindungen gemäss Anspruch 1 oder 2 der Formel

(7)

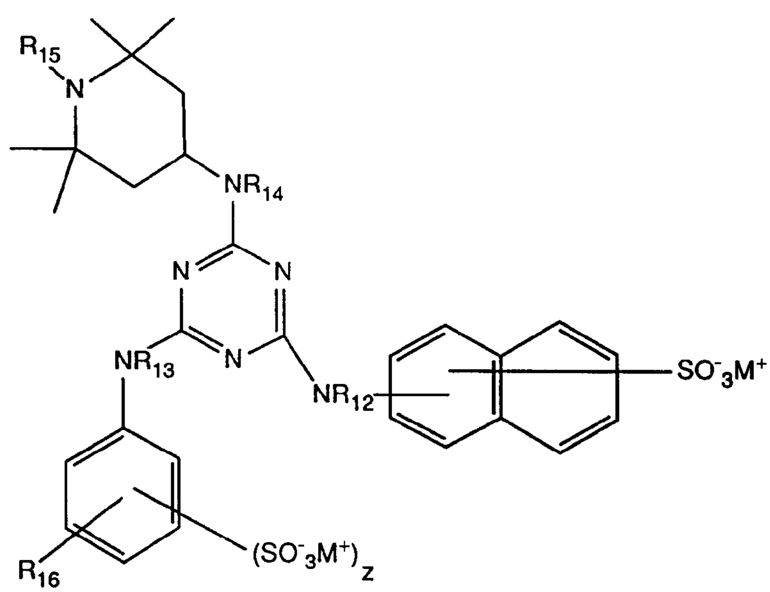

worin
$R_{12}$, $R_{13}$, $R_{14}$ und $R_{15}$ unabhängig voneinander, Wasserstoff oder $C_1$-$C_5$Alkyl,
$R_{16}$ Wasserstoff, Halogen, $C_1$-$C_5$Alkyl, $C_1$-$C_5$Alkoxy, Carboxy, Carboxy-$C_1$-$C_5$Alkyl, Mono- oder Di-$C_1$-$C_5$Aylamino,
M unabhängig voneinander Wasserstoff oder Alkalimetall und

z 0 oder 1 bedeuten.

**7.** Verbindungen gemäss Anspruch 1 oder 2 der Formel

(8)

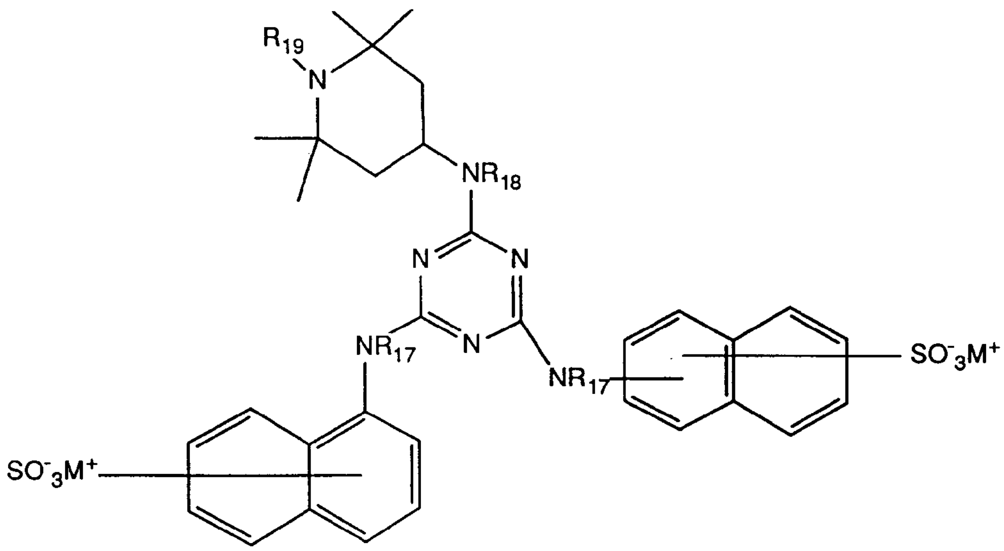

worin

$R_{17}$, $R_{18}$ und $R_{19}$ unabhängig voneinander Wasserstoff oder $C_1$-$C_5$Alkyl und

M unabhängig voneinander Wasserstoff oder Alkalimetall bedeuten.

**8.** Verbindungen gemäss Anspruch 1 oder 2, worin in Formel (1)

$R_1$ einen Rest der Formel

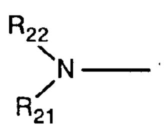

bedeutet, wobei

$R_{21}$ und $R_{22}$ unabhängig voneinander, Wasserstoff, $C_1$-$C_5$Alkyl, Cycloalkyl, unsubstituiertes oder durch $C_1$-$C_5$Alkyl substituiertes Phenyl oder

$R_1$ 1-Azacycloalkyl oder Morpholino bedeutet.

**9.** Verfahren zur Herstellung der Verbindungen gemäss Formel (1), dadurch gekennzeichnet, dass man eine Verbindung der Formel

(9)

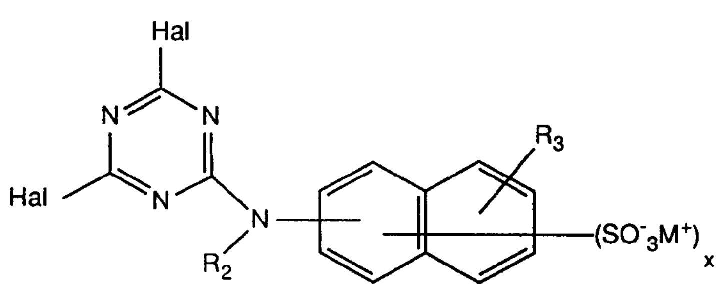

mit einem Mol der Verbindung der Formel

(4a) 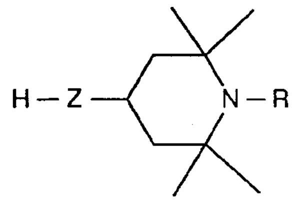 oder

die Verbindung der Formel (9) mit 1 Mol der Verbindung der Formel

(2a) 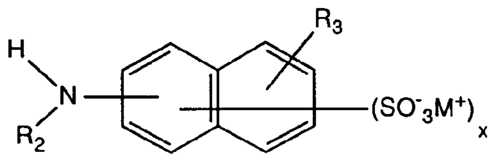

und 1 Mol der Verbindung der Formel (4a) oder
die Verbindung der Formel (9) mit 2 Mol der Verbindung (4a) oder die Verbindung der Formel (9) mit einem Mol einer $C_1$-$C_5$Alkanolat-, Cycloalkanolat-, Phenyl-$C_1$-$C_5$Alkanolat-, Phenolat-, $C_1$-$C_5$Alkylthiolat-, Cycloalkylthiolat- oder einer Phenylthiolatverbindung, eines Mono- oder Di-$C_1$-$C_5$Alkylamins, Mono- oder Di-$C_1$-$C_5$Alkylamins, Di-$C_3$-$C_5$Alkenylamins, Cycloalkylamins, Phenylamins, einer 1-Azacycloalkyl-, einer Morpholinoverbindung und 1 Mol der Verbindung (4a) oder die Verbindung der Formel (9) mit einem Mol der Verbindung der Formel

(3a) 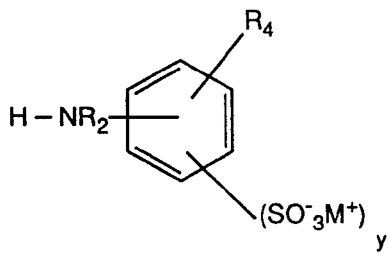

und einem Mol der Verbindung der Formel (4a) in beliebiger Reihenfolge umsetzt, wobei

R Wasserstoff, Oxyl, Hydroxy, $C_1$-$C_5$Alkyl, $C_3$-$C_5$Alkenyl, $C_1$-$C_5$Alkoxy, Acyl oder Benzyl,
$R_2$ Wasserstoff oder $C_1$-$C_5$Alkyl,
$R_3$ Wasserstoff oder Hydroxy,
$R_4$ Wasserstoff, Halogen, $C_1$-$C_5$alkyl, $C_1$-$C_5$Alkoxy, Carboxy, Carboxy-$C_1$-$C_5$Alkyl oder Mono- oder Di-$C_1$-$C_5$-Acylamino,
M gleich oder verschieden sein können und Wasserstoff, Alkalimetall, Erdalkalimetall, Ammonium oder einen organischen Ammoniumrest
Z -O- oder -($NR_5$)-,
worin $R_5$ Wasserstoff oder $C_1$-$C_5$Alkyl,
Hal ein Halogenatom,
x 1 oder 2 und
y 0 oder 1 bedeutet.

**10.** Verfahren zur Herstellung der Verbindungen der Formel (1) worin
$R_1$ $C_1$-$C_5$Alkyl oder Phenyl ist, dadurch gekennzeichnet, dass man 1 Mol einer 2,4-Dihalogen-6-$C_1$-$C_5$Alkyl- bzw. 6-phenyl-s-triazinverbindung nacheinander mit einem Mol der Verbindung der Formel (2a) und 1 Mol der Verbindung der Formel (4a) umsetzt.

**11.** Verfahren zur Herstellung der Verbindungen der Formel (5) gemäss Anspruch 4, dadurch gekennzeichnet, dass man 1 Mol der Verbindung der Formel (9) mit einem Mol der Piperidinverbindung der Formel (4a) umsetzt.

**12.** Verfahren zur Herstellung der Verbindungen der Formel (6) gemäss Anspruch 5, dadurch gekennzeichnet, dass man 1 Mol der Piperidinverbindung der Formel (4a) mit 1 Mol der Verbindung der Formel (5) umsetzt.

**13.** Verfahren zur Herstellung der Verbindungen der Formel (6) gemäss Anspruch 5, dadurch gekennzeichnet, dass man 1 Mol der Verbindung der Formel (9) mit 2 Mol der Piperidinverbindung der Formel (4a) umsetzt.

**14.** Verfahren zur Herstellung der Verbindungen der Formel (7) gemäss Anspruch 6, dadurch gekennzeichnet, dass man 1 Mol der Verbindung der Formel (9) mit 1 Mol der Verbindung der Formel (3a) und 1 Mol der Verbindung der Formel (4a) umsetzt.

**15.** Verfahren zur Herstellung der Verbindungen der Formel (8) gemäss Anspruch 7, dadurch gekennzeichnet, dass man ein Mol der Verbindung der Formel (9) nacheinander mit einem Mol einer Aminonaphthalinsulfonsäure der Formel (2a) und 1 Mol der Piperidinverbindung der Formel (4a) umsetzt.

**16.** Verfahren zur Herstellung der Verbindungen der Formel (1), worin
$R_1$ ein Rest der Formel

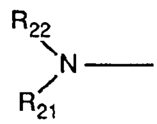

ist gemäss Anspruch 8, dadurch gekennzeichnet, dass man eine Verbindung der Formel (9) mit einer Piperidinverbindung der Formel (4a) und der entsprechenden N-Alkylverbindung oder Aminophenylverbindung der Formel

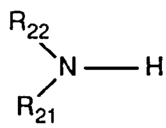

umsetzt.

**17.** Verfahren zur photochemischen und thermischen Stabilisierung von Polyamidfasermaterialien, dadurch gekennzeichnet, dass man gefärbte oder ungefärbte Polyamid-Fasermaterialien mit wasserlöslichen Triazinderivaten der Formel

(1) 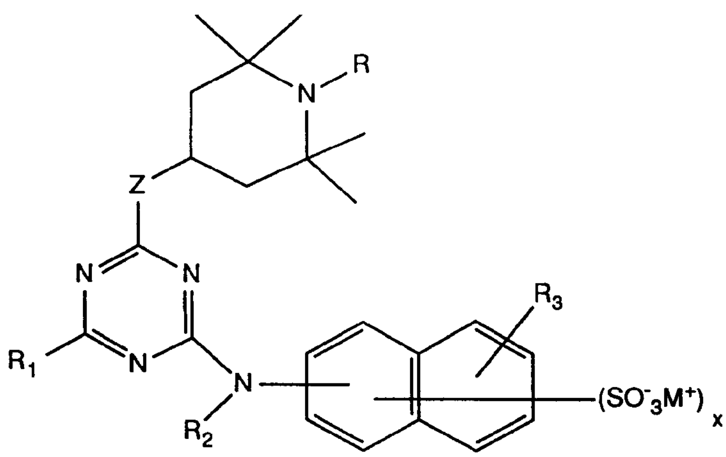

behandelt, worin
R Wasserstoff; Oxyl; Hydroxy; $C_1$-$C_5$Alkyl; $C_3$-$C_5$Alkenyl; $C_1$-$C_5$Alkoxy; Acyl; oder Benzyl
$R_1$ Halogen; $C_1$-$C_5$Alkyl; Amino; $C_1$-$C_5$Alkoxy; $C_3$-$C_5$Alkenyloxy; Cycloalkoxy; nicht substituiertes

oder im Phenylteil durch Halogen, $C_1$-$C_5$Alkyl, $C_1$-$C_5$Alkoxy, Carboxy, Carboxy-$C_1$-$C_5$Alkyl, Carbamoyl, Mono- oder Di-$C_1$-$C_5$-Acylamino oder Acyl substituiertes Phenoxy; Phenyl; Phenyl-$C_1$-$C_5$Alkyl; Phenylthio; Phenyl-$C_1$-$C_5$Alkylthio; Mono- oder Diphenyl-$C_1$-$C_5$Alkylamino; $C_1$-$C_5$Alkylthio; Cycloalkylthio; nicht substituiertes oder durch Hydroxy oder Carboxy substituiertes Mono- oder Di-$C_1$-$C_5$Alkylamino, wobei die Alkylkette durch ein Sauerstoffatom unterbrochen sein kann; Mono- oder Di-$C_3$-$C_5$Alkenylamino; nicht substituiertes oder durch $C_1$-$C_5$Alkyl substituiertes Mono- oder Dicycloalkylamino; nicht substituiertes oder durch $C_1$-$C_5$Alkyl, Hydroxy oder Carboxy substituiertes 1-Azacycloalkyl; nicht substituiertes oder durch $C_1$-$C_5$Alkyl substituiertes Morpholino; einen Rest der Formel

(2) 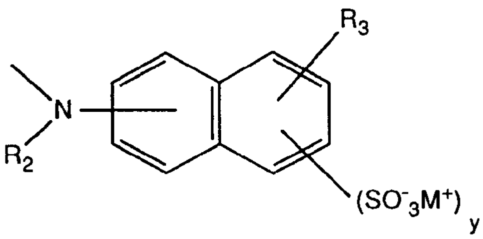 ,

einen Rest der Formel

(3) 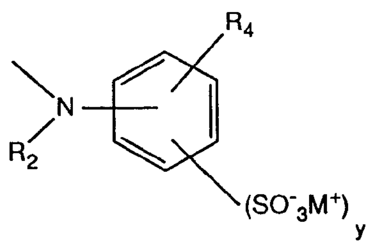

oder einen Rest der Formel

(4) 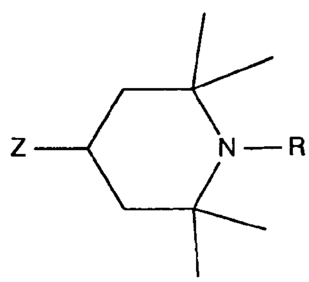

| | |
|---|---|
| $R_2$ | Wasserstoff; oder $C_1$-$C_5$Alkyl, |
| $R_3$ | Wasserstoff; oder Hydroxy, |
| $R_4$ | Wasserstoff; Halogen; $C_1$-$C_5$Alkyl; $C_1$-$C_5$Alkoxy; Carboxy; Carboxy-$C_1$-$C_5$Alkyl; Acyl; Carbamoyl; oder Mono- oder Di-$C_1$-$C_5$-Acylamino, |
| M | gleich oder verschieden sein können und Wasserstoff; Alkalimetall; Erdalkalimetall; Ammonium; oder einen organischen Ammoniumrest der Formel $(C_1$-$C_4Alkyl)_n(H)_mN^+$; und |
| Z | -O-; oder -($NR_5$)-, |
| $R_5$ | Wasserstoff; oder $C_1$-$C_5$Alkyl, |
| m | 0 bis 3; |
| n | 1 bis 4; und die Summe m + n = 4 |
| x | 1 oder 2 und |
| y | 0 oder 1,bedeuten |

und, wenn $R_1$ einen Rest der Formel (2) oder (3) bedeutet und y 1 ist, x in Formel (1) 1 ist.

Europäisches Patentamt

# EUROPÄISCHER RECHERCHENBERICHT

Nummer der Anmeldung
EP 92 81 0932

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | CHEMICAL ABSTRACTS, vol. 115, 1991, Columbus, Ohio, US; abstract no. 73798u, TAKIMOTO, HIROSHI ET AL. 'Inks for jet printing and writing apparatus' Seite 116 ; * Zusammenfassung * & JP 91 39,372 (Mitsubishi Kasei Corp.) --- | 1,17 | C07D401/12<br>C08K5/3492<br>C07D401/14 |
| A | EP-A-0 194 885 (SUMITOMO CHEMICAL CO., LTD) * Seite 1-4 * --- | 1 | |
| Y | CHEMICAL ABSTRACTS, vol. 105, 1986, Columbus, Ohio, US; abstract no. 116416f, B.A. KOROLEV ET AL. 'Synthesis and physicochemical properties of reactive monochlorotriazine light stabilizers and reactive dyes containing a 4-amino-2,2,6,6-tetrametylpiperidine radical' Seite 70 ; * Zusammenfassung * & Zh. Prikl. Khim. 1986,59(5),1144-7 --- | 1,17 | RECHERCHIERTE SACHGEBIETE (Int. Cl.5)<br>C07D |
| A | EP-A-0 062 825 (BAYER AG) * Seite 1,4(Zeile 3-8), 11(Zeile 1-14 ) * --- | 1 | |
| A | EP-A-0 453 405 (CIBA-GEIGY AG) * Anspruch 1; Seite 33, Zeile 6-14 * --- | 1 | |
| A | FR-A-2 521 143 (APITAL PRODUZIONI INDUSTRIALI SPA) * Ansprüche * ----- | 1 | |

Der vorliegende Recherchenbericht wurde für alle Patentansprüche erstellt

| Recherchenort | Abschlußdatum der Recherche | Prüfer |
|---|---|---|
| DEN HAAG | 25 JANUAR 1993 | VAN BIJLEN H. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)